# EUROPEAN PATENT APPLICATION

(11) **EP 2 487 183 A1**
(43) Date of publication of application: **15.08.2012**
(21) Application number: 11000351.4
(22) Date of filing: 18.01.2011
(51) Int. Cl.: C07K 7/02, C07K 14/00

(54) **Ubiquitin-isopeptide probes**

(71) Applicant: Helmholtz-Zentrum für Infektionsforschung GmbH, 38124 Braunschweig (DE)
(72) Inventor: Iphöfer, Alexander, 38100 Braunschweig (DE); Franke, Raimo, 38120 Braunschweig (DE); Ritter, Antje, 38239 Salzgitter (DE); Arnold, Tatjana, 38124 Braunschweig (DE); Jänsch, Lothar, 38302 Wolfenbüttel (DE)
(74) Representative: Forstmeyer, Dietmar

(57) **Abstract**

The invention relates to a ubiquitin-isopeptide probe (hereinafter also referred to as UIPP), a method for its preparation, and its use. The invention also provides a method for isolating a deubiquitinating enzyme and a method for activity-based protein profiling (ABPP).

## Description

### Field of the Invention

The invention relates to an ubiquitin-isopeptide probe (hereinafter also referred to as UIPP), a method for its preparation, and its use. The invention also provides a method for isolating a deubiquitinating enzyme and a method for activity-based protein profiling (ABPP).

### Background of the Invention

Ubiquitin (Ub) is a 76 amino acid small protein. Many biochemical pathways are regulated in part by posttranslational modification of proteins with Ub and ubiquitin-like (UBL) molecules. This posttranslational protein modification by Ub — a process known as ubiquitination or ubiquitylation — is, for example, involved in the regulation of biological processes such as protein degradation, gene transcription, cell-cycle progression, DNA repair, apoptosis, virus budding and receptor endocytosis. Precise regulation is achieved through the opposing actions of Ub/UBL-specific conjugating and deconjugating enzymes.

Ub/UBL can be conjugated to target proteins either as a monomer or as Ub chains that vary in length and linkage type. The various types of Ub/UBL modification are linked to distinct physiological functions in cells. Possible protein modifications by Ub have been recently reviewed by Ikeda and Dikic (EMBO Rep. 2008; 9(6): 536―542; doi: 10.1038/embor.2008.93).

There are predicted to be nearly 100 Ub specific proteases encoded by the human genome. These Ub specific proteases themselves are regulated by posttranslational modifications leading to specificity in substrate selection. Given the breadth of processes that involve modification by Ub or UBLs, it is not surprising that malfunction of several members is known to cause diseases. Deubiquitinating enzymes (DUBs), including UBL-specific proteases (ULPs), deconjugate substrates modified with Ub/UBLs and recycle Ub/UBL inside the cell. Human DUBs can be divided into five subfamilies: ubiquitin-specific proteases (Usp), ubiquitin carboxy-terminal hydrolases (UCH), ovarian tumour-like proteases (OTU), JAMM/MPN metalloproteases, and Machado-Jakob-disease proteases (MJD). An overview of the current understanding of structure and function of DUBs is, for example, presented by Komander, D., Clague, M.J., and Urbé, S. in Nat. Rev. Mol. Cell. Biol. 2009; 10: 550-63. Apart from the JAMM/MPN family members, which are zinc metalloproteases, all other DUBs are cystein proteases. Most DUBs catalyse the hydrolysis of the isopeptide bond between a Lys ε-amino group and a carboxyl group corresponding to the C terminus of ubiquitin. DUBs and ULPs contribute to the regulation and fine tuning of several signaling events by removing covalently attached Ub/UBL from proteins. Moreover DUBs are involved in host-pathogen interactions, and it has already been shown that certain bacteria encode their own DUBs which act as virulence factors. Therefore, DUBs have also emerged as promising therapeutic targets in several diseases such as, cancer, neurodegenerative diseases, inflammatory diseases, and viral or bacterial infections.

The characterization of the DUBs by standard proteomics methods is hampered by the fact that their expression level is typically very low. Moreover, the expression level only shows the existence but not the enzymatic activity of the DUB, which is mostly regulated by posttranslational modifications. The presence or absence of enzymatic activity, however, is ultimately the most relevant information when studying biochemical pathways. Activity-based protein profiling (ABPP) has emerged as a powerful chemical proteomic strategy which allows to characterize enzyme function directly in native biological systems on a global scale. The basic technology of ABPP, the enzyme classes addressable by this method, and the biological discoveries attributable to its application have been recently reviewed by Cravatt et al. (Annu. Rev. Biochem. 2008; 77: 383-414). An advantage of ABPP over traditional techniques is the applicability to a cell or tissue of choice and that molecular imaging and pharmacology applications are possible.

ABPP relies on the design of active-site directed covalent probes to interrogate specific subsets (families) of enzymes in complex proteomes and to provide the basis for a quantitative readout of the functional state of individual enzymes in the family. Prototypic ABPP probes target a large, but manageable, fraction of the enzyme proteome, often defined by shared catalytic features. The size of the enzyme subset, depends on the specificity and can range from a few proteins to several hundred. ABPP probes utilize a range of chemical scaffolds, including mechanism-based inhibitors, protein-reactive natural products, and general electrophiles. Activity-based probes (irreversible covalent inhibitors with reporter groups) for identification and mechanistic study of DUBs/ULPs by ABPP are known. These activity-based probes (ABPs) have been developed to selectively target only DUBs which are enzymatically active at a certain time point. Specifically, the ABPs allow the characterization of the otherwise not accessible DUB sub-proteome and the identification of previously unknown DUBs. The ABPs can also be used as molecular probes to investigate DUBs and the regulation of the ubiquitin system in infection processes or serve as drug lead structures for novel therapeutics.

For instance, Love et al. (Chem Biol. 2009; 4: 275-287; doi:10.1021/cb9000348) disclose Ubiquitin C-terminal electrophiles as Ub-based active site-directed probes which were used to identify novel DUBs/ULPs by ABPP. Borodovsky et al. (Chem Biol 2002; 9; 1149-1159) report the synthesis of HAUb-derived probes specific for the DUB superfamily and show that 23 DUBs are targeted by these probes in EL4 cell lysates, including 10 polypeptides for which no enzymatic activity had been previously demonstrated thereby demonstrating that novel members of an enzyme family can be identified by this approach. A thorough overview of ABPs of Ub and UBLs in the identification, activity profiling, and structural analysis of Ub/UBL-specific proteases is given by Galardy et al. (Methods Enzymol. 2005; 399: 120-131; DOI: 10.1016/S0076-6879(05)99008-3).

Drugs that inhibit the catalytic activity of specific DUBs hold great promise for modulating the ubiquitin-dependent physiological processes that are involved in human disease (Wilkinson, K.D. 2009; J. Cell Sci. 122, 2325-9).

However, despite the importance of DUBs/ULPs in health and disease, relatively few tools have been generated to facilitate their biochemical analysis.

Thus, although a number of ABPs relating to DUBs/ULPs exist or are currently under development, it has so far not been possible to target all putative DUBs/ULPs. Additionally, currently employed ABPs do not target DUBs with a distinct specificity for a certain substrate or poly-Ub linkage. The previously introduced ABPs only exhibit a broad general reactivity towards DUBs, but are not able to target a certain subgroup or single DUBs with a particular specificity, such as specificity for K48-linked poly-Ub chains. As a result, a need remains to provide ABPs that may be used to target DUBs/ULPs with distinct specificity for a target protein or a poly-Ub linkage.

It is, therefore, an object of the present invention to provide a novel Ub-isopeptide probe (UIPP) that enables specific covalent capture of DUBs/ULPs. Preferably, the UIPP according to the invention allows identification and characterization of new DUBs/ULPs or the identification and characterization of previously unknown substrate specificities / mode of action of known DUBs/ULPs.

### Summary and Description of the Invention

The present invention was made in view of the prior art and the needs described above, and, therefore, the object of the present invention is to provide a novel alternative UIPP enabling to capture DUBs with distinct target specificity and a method for its preparation.

Other objects of the present invention are the use of the UIPP according to the invention, a method for isolating a deubiquitinating enzyme and a method for ABPP of DUBs/ULPs.

These objects are solved by the subject matter of the attached claims.

These and other aspects of the present invention will become apparent upon reference to the following detailed description and definitions.

The present invention relates to an ubiquitin-isopeptide-probe (UIPP), which probe comprises a branched amino acid sequence. Specifically, the UIPP of the present invention comprises a peptide sequence, characterized in that the sequence contains one or more residue(s) X, wherein X is represented by formula (1): 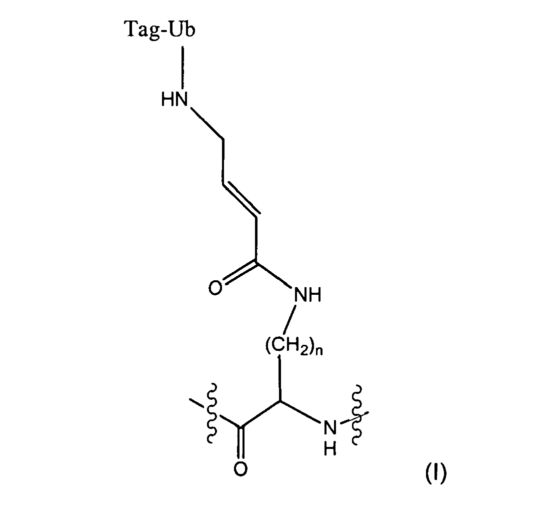 wherein
Tag is a first reporter tag that is N-terminally attached to Ub;
Ub is ubiquitin(1-75) (also referred to as Ub₁₋₇₅) or a fragment of Ub₁₋₇₅ that comprises at least residues 66 to 75 of Ub₁₋₇₅; and
n is 1, 2, 3 or 4.

Ubiquitin(1-75) or Ub refers to residues 1 to 75 of the small protein ubiquitin, i.e. ubiquitin which lacks the C-terminal Gly76. The sequence of human ubiquitin (1-75) in one-letter code (lysine residues in bold) is as follows:

MQIFVKTLTGKTITLEVEPSDTIENVKAKIQDKEGIPPDQQRLIFAGKQLEDGRTLSDYNIQKE
STLHLVLRLRG.

A fragment of Ub₁₋₇₅ that comprises at least residues 66 to 75 of Ub₁₋₇₅ as used herein denotes a N-terminally truncated version of ubiquitin(1-75), i. e. a polypeptide that consists at least of residues 66 to 75 of Ub₁₋₇₅.

The term "Tag" or "reporter tag" as used herein denotes a biochemical marker or label, i.e. an easily recognizable chemical moiety, e.g. a protein, peptide, or small molecule, that is covalently attached to the N-, or C-terminus, preferably the N-terminus of a protein, polypeptide or peptide sequence. Numerous such protein labels exist and are known and commonly employed by those skilled in the art. Examples for a reporter tag are affinity labels, e.g. affinity tags (Kimple and Sondek, BioTechniques (2002), 33:578-590), fluorophores, biotin, or radioactive label.

The terms "protein", "polypeptide", "peptide" and "peptide sequence" as used herein define an organic compound made of two or more amino acid residues arranged in a linear chain, wherein the individual amino acids in the organic compound are linked by peptide bonds, i.e. an amide bond formed between adjacent amino acid residues.

The UIPP of the invention allows identification and characterization of new DUBs/ULPs or the identification and characterization of previously unknown substrate specificities / mode of action of known DUBs/ULPs. The main characterisitic of the UIPP that enables this selectivity is the fact that it is a true substrate mimetic. This is, contrary to hitherto known ABPs, the UIPP according to the invention contains a ubiquitine, which is C-terminally linked via an isopeptide bond to a synthetic peptide. The peptide sequence can originate from a second ubiquitine unit or a target protein sequence. The isopeptide linker contains a electrophilic reactive group that enables covalent capture of the DUB. Accordingly, the UIPP of the invention does not only enable selective capture of DUBs but also allows elucidation of their distinct mechanism of action and their target specificity. The UIPP furthermore facilitates crystallisation of DUBs in complex with the ABP, i.e. UIPP, and subsequent structure determination. The specificity of the UIPP for a subgroup of DUBs of even single DUBs in a particular posttranslationally modified state also makes it an attractive drug lead for the selective inhibition of DUBs.

Preferably, the peptide sequence of the UIPP according to the invention is derived from ubiquitin, or from a target protein with ubiquitination site. Examples of target proteins with ubiquitination site are E3 ubiquitin-protein ligase Mdm2, phosphatidylinositol-3,4,5-trisphosphate 3-phosphatase and dual-specificity protein phosphatase PTEN, Myc proto-oncogene protein, breast cancer type 1 susceptibility protein BRACA1, and hypoxia-inducible factor 1-alpha HIF 1 α.

Especially preferred, the peptide sequence of the UIPP according to the invention comprises a motif selected from the group: FAGXQLE, NIQXEST, IFVXTLT, LTGXTIT, ENVXAKI, ENVXAKIQD, ENVKAXIQD, ENVXAXIQD, IQDXEGI, GSDQXDLVQ, LQEEXPSSS, DCKXTIVND, DCXKTIVND, ENDDXITQ, SQEDVXEFE, DXEESVEEE, ISEXAKLEN, ISEKAXLEN, LYFTXTVEE, FKVXLYFTK, EETSEXVEN, SSPXSCAS, VVSVEXRQA, TEENVXRRT, PEHLXDEVS, NTTEXRAAE, RHPEXYQGS, STEKXVDLN, PSTEXKVDL, ESNAXVADV, ENKTXGDSI, HENXTKGDS, IQNEXNPNP, EQTSXRHDS, AEDPXDLML, ESNIXPVQT, ENVFXEASS, DGEIXEDTS, ENDIXESSA, LSLXNSLND, VILAXASQE, SXQMRHQGS, STSEXAVLT, LTSQXSSEY, LSADXFEVS, STSXNKEPG, LSADXFEVS, TSKNXEPGV, VPQLXVAES, VSREXPELT, QLFTXVESE, SPNEXLQNI, AETPXPLRS, EVALKLEPN, DTEAXNPFS, TDELXTVTK, KTVTKDRME, THIHXETTS, and EQTEXSHPR.

Further preferred is an UIPP according to the invention, wherein the peptide sequence further comprises a second reporter tag.

Particularly preferred is an UIPP according to the invention, wherein the first and the second reporter tag are each and independently of each other selected from an affinity tag, e.g. a tag derived from hemagglutinin (HA-tag; N-YPYDVPDYA-C), polyhistidine tag (His₅₋₁₀, preferably His₆; also referred to as His-tag), N-DYKDDDDK-C (1012 Da; also referred to as FLAG-tag), N-EQKLISEEDL-C (1202 Da; also referred to as Myc-tag); biotin; or a fluorophore.

Especially preferred is an UIPP, wherein the first reporter tag is HA-tag, His-tag, FLAG-tag, Myc-tag, biotin, or a fluorophore.

Also preferred is an UIPP, wherein the second reporter tag is HA-tag, His-tag, FLAG-tag, Myc-tag, biotin or a fluorophore.

Preferably, the first and the second reporter tag are the same in an UIPP according to the invention.

Further preferred is an UIPP, wherein the peptide sequence is selected from the group: RLIFAGXQLEDGR (Ub₄₂₋₅₄); YPYDVPDYARLIFAGXQLEDGR (HA-Ub₄₂₋₅₄); RTLSDYNIQXESTLHLVLR (Ub₅₄₋₇₂); and YPYDVPDYARTLSDYNIQXESTLHLVLR (HA-Ub₅₄₋₇₂).

Especially preferred is an UIPP, wherein n is 3 or 4.

The present invention also encompasses a method for preparing an UIPP according to the invention, wherein the method comprises the steps of:
(a) synthesis of a peptide that comprises at least one lysine, ornithine, 2,3-diaminopropionic acid, or 2,4-diaminobutyric acid residue, wherein the amino group in the side chain of the lysine, ornithine, 2,3-diaminopropionic acid, or 2,4-diaminobutyric acid residue is orthogonally protected by a protecting group selected from: Alloc (allyloxycarbonyl), Dde 1-(4,4-dimethyl-2,6-dioxocyclohex-1-ylidene) ethyl, ivDde (1-(4,4-dimethyl-2,6-dioxocyclohex-1-ylidene)-3-methylbutyl), Mmt (methoxytrityl), Mtt (methyltrityl), and Z (benzoyloxycarbonyl);
(b) reacting the peptide obtained in step (a) with N-tert-butyloxycarbonyl-(E)-4-amino-2-butenoic acid according to formula (II) 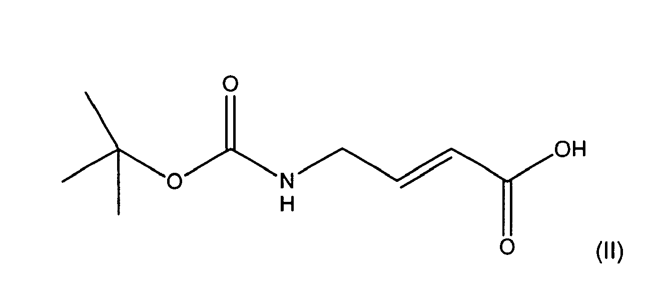 to thereby form a side chain modified peptide comprising at least one residue Y, wherein Y is represented by formula (III) 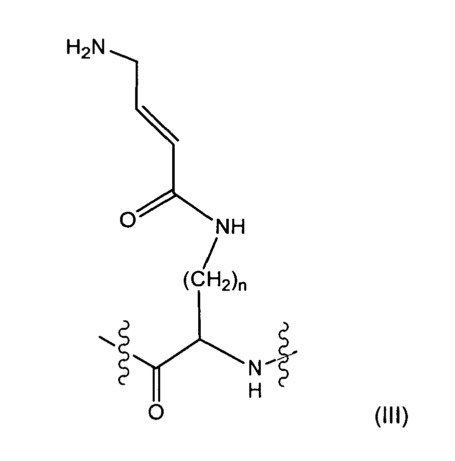 wherein n is 1, 2, 3 or 4.
(c) intein-based chemical ligation between the side chain modified peptide of step (b) and a modified ubiquitin thioester to thereby form the ubiquitin-isopeptide-probe according to the present invention.

Preferably, the modified ubiquitin thioester used in step (c) of the method according to the invention is HA-Ub₇₅-MESNa thioester.

Especially preferred step (c) of the method according to the invention is carried out by mixing purified HA-Ub₇₅-MESNa thioester dissolved in 2-[4-(2-hydroxyethyl)piperazin-1-yl]ethanesulfonic acid (HEPES) buffer pH 7 with Sulfo-NHS (N-hydroxysulfosuccinimide), followed by adding the side chain modified peptide of step (b) to the reaction solution, adjusting the pH of the reaction solution to pH 8 using Na₂C0₃ or NaOH, forming the UIPP by ligating the HA-Ub₇₅-MESNa thioester and the side chain modified peptide overnight at 37 °C, dialyzing the reaction solution containing the ligation product, and ultrafiltrating the resulting dialysis solution containing the ligation product using a membrane having a molecular weight cut off (MWCO) of 500-5000 Da.

The present invention further provides a method for isolating a deubiquitinating enzyme (DUB) from a sample containing cells or a cell extract, wherein the method comprises the steps of:
(a) treating the sample with an UIPP according to the invention; and
(b) separating DUBs.

Preferably, separation of DUBs according to step (b) of the method for isolating a DUB is carried out using magnetic separation, immunological separation, gel filtration chromatography, affinity chromatography, column chromatography, displacement chromatography, electro chromatography, gas chromatography, high performance liquid chromatography, ion chromatography, micellar electrokinetic chromatography, normal phase chromatography, paper chromatography, reversed-phase chromatography, size exclusion chromatography, thin layer chromatography, gel electrophoresis, centrifugation, adhesion, or flow cytometry.

The UIPP according to the present invention can be used as a research tool, e.g. as molecular probes to investigate DUBs and the regulation of the ubiquitin system in infection processes and furthermore serve as drug lead structure for novel therapeutics.

Preferably, the UIPP according to the invention can be used to identify ubiquitination sites of target proteins or to detect, purify and/or identify deubiquitinating enzymes (DUBs) with a specificity for a certain target protein or poly-Ub linkage.

The present invention also provides a method for deubiquitinating enzyme analysis of a DUB or a DUB-UIPP-protein complex isolated from a sample containing cells or a cell extract using an UIPP according to the invention, the method comprising the steps of:
(a) tryptic digestion of the isolated DUB or DUB-UIPP-protein complex; and
(b) analysing the product of the tryptic digestion of step (a) by liquid chromatography coupled to mass spectrometry (LC/MS).

The therapeutic use of the UIPP according to the present invention as a medicament, or of its pharmacologically acceptable salts, prodrugs, solvates and hydrates and also formulations and pharmaceutical compositions containing the same are within the scope of the present invention. The present invention also relates to the use of these compounds as active ingredient(s) in the preparation or manufacture of a medicament, especially, the use of an UIPP of the invention, its pharmacologically acceptable salts, prodrugs or solvates and hydrates and also formulations and pharmaceutical compositions for the treatment of cancer, neurodegenerative disorders, inflammatory diseases, cystic fibrosis, and viral or bacterial infections.

The pharmaceutical compositions according to the present invention comprise at least one compound of formula (I), (II) or (III) and, optionally, one or more carrier substances, excipients and/or adjuvants. Pharmaceutical compositions may additionally comprise, for example, one or more of water, buffers such as, e.g., neutral buffered saline or phosphate buffered saline, ethanol, mineral oil, vegetable oil, dimethylsulfoxide, carbohydrates such as e.g., glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical compositions provided herein. For instance, the compounds of the invention may advantageously be employed in combination with an antibiotic, anti-fungal, or anti-viral agent, an-anti histamine, a non-steroidal anti-inflammatory drug, a disease modifying anti-rheumatic drug, a cytostatic drug, a drug with smooth muscle activity modulatory activity or mixtures of the aforementioned.

Pharmaceutical compositions may be formulated for any appropriate route of administration, including, for example, topical such as, e.g., transdermal or ocular, oral, buccal, nasal, vaginal, rectal or parenteral administration. The term parenteral as used herein includes subcutaneous, intradermal, intravascular such as, e.g., intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate.

For the preparation of such tablets, pills, semi-solid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

For the treatment of microbial or fungal infections as well as cancer, the dose of the biologically active compound according to the invention may vary within wide limits and may be adjusted to individual requirements. Active compounds according to the present invention are generally administered in a therapeutically effective amount. Preferred doses range from about 0.1 mg to about 140 mg per kilogram of body weight per day, about 0.5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination, i.e. other drugs being used to treat the patient, and the severity of the particular disease undergoing therapy.

It is to be noted that the present invention also encompasses all possible combinations of all preferred embodiments.

### Brief description of the figures

***Figure 1******.*** Schematic representation of an UIPP according to the present invention.
***Figure 2******.*** Results of an activity-based DUB assay employing an UIPP comprising peptide sequence HA-Ub₄₂₋₅₄, an UIPP comprising peptide sequence HA-Ub₅₄₋₇₂, and commercially available probe HAUb-VME as positive control are shown. The possibilty to selectively capture known DUBs like UCH L-3 (preference for K48-linked poly-Ub) and USP15 (preference for both K48- and K63-linked poly-Ub) with a distinct subtrate specificity is demonstrated by the observed band shift resulting from the protein complex formed between the DUB and UIPP.

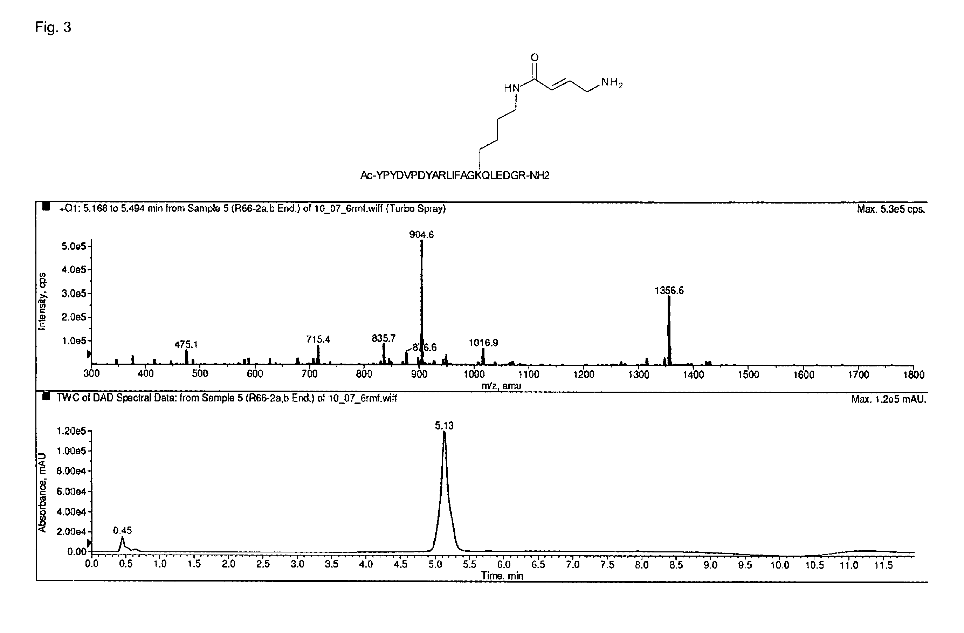

***Figure 3******.*** This figure shows the chemical structure of modified peptide HA-Ub₄₂₋₅₄, its mass spectrum and LC chromatogram.

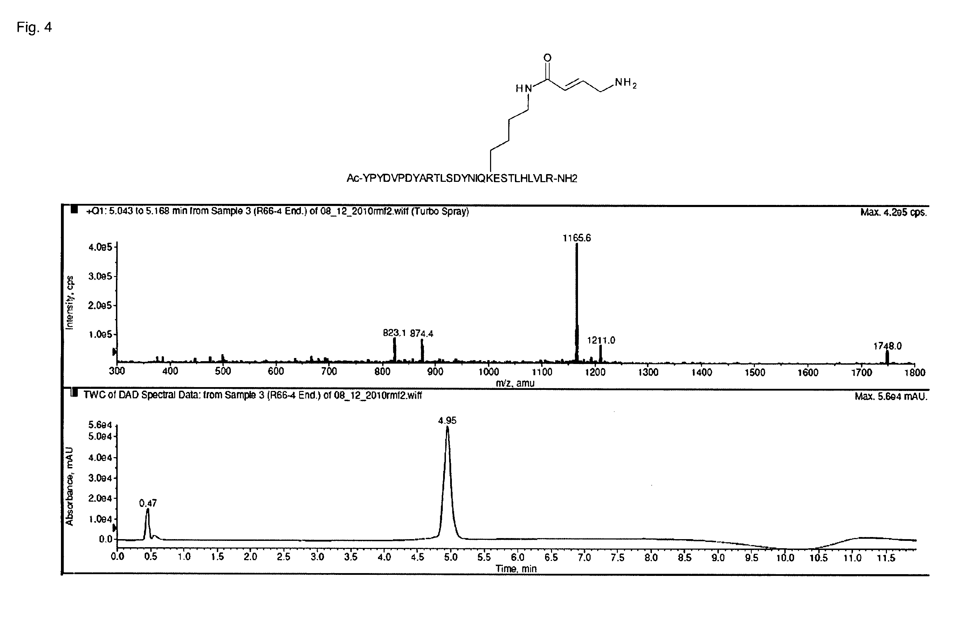

***Figure 4******.*** This figure shows the chemical structure of modified peptide HA-Ub₅₄₋₇₂, its mass spectrum and LC chromatogram.

### Examples

### 1. Synthesis of activity- based ubiquitin-isopeptide probe (UIPP)

### Preparation of N-tert-butyloxycarbonyl (Boc)-glycinal (2)

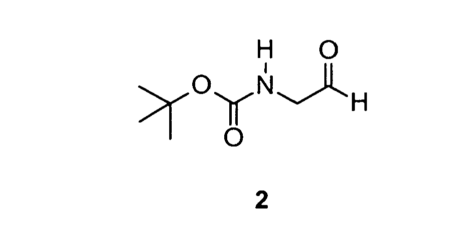

Triethylamine ( 7.8 ml, 55.8 mmol) was added to a solution of N-Boc-glycinol (3 g, 18.6 mmol) in dichloromethane (60 ml) at 0°C. SO₃-pyridine (8,9 g, 55,8 mmol) was first dissolved in DMSO and pyridine (0.24 ml, 2.97 mmol) was added. After 10 minutes the SO₃-pyridine in DMSO was added dropwise to the N-Boc-glycinol solution at 0°C, upon which the solution turned from clear to yellow. The ice bath was removed and the solution stirred for further 30 min at room temperature. The reaction mixture was poured into 180 ml ice could brine. After removal of the dichloromethane layer, the aqueous layer was extracted with diethyl ether (3x120 ml). The combined organic layers were washed with ice cold NaHSO₄ solution (1 M, 1 x 42 ml) and with ice cold brine (2 x 42 ml). The organic layer was dried over MgSO₄ anhydrous, filtered and concentrated under vacuum. A silica gel column was used to purify N-tert-butyloxycarbonyl (Boc)-glycinal (2). Aldehyde (2) was eluted with 20% ethyl acetate in dichloromethane and isolated with 68% yield (2.0 g, 12.6 mmol).

### Preparation of N-tert-butyloxycarbonyl-(E)-4-amino-2-butenoic acid (3)

n-Buthyllithium (4.8 ml, 1.6 M in hexane, 7.54 mmol) was dissolved in THF (12 ml) at -78°C. Diethyl phosphono acetic acid is dissolved in THF (5 ml) and added dropwise to the nBuLi solution at -78°C. The solution turned slightly yellow and was stirred for 30 min at -78°. N-tert-butyloxycarbonyl (Boc)-glycinal (2) was dissolved in THF (3 ml) and added dropwise to the solution at -78°C. After stirring for 3h at room temperature water (12 ml) was added to the solution. The organic layer was separated and washed with 10% NaHCO₃ (2 x 10 ml), the combined aqueous layers were then acidified to pH 3.5 with conc. HCl and extracted with diethyl ether (3 x 18 ml). The combined organic layers were dried over MgSO₄ anhydrous, filtered and concentrated to yield compound 3, which was further purified using silica gel chromatography. Compound 3 was eluted with 20% ethyl acetate in dichloromethane containing 1% acetic acid to yield 389 mg (1.93 mmol, 51%) of the purified compound 3. Reaction Scheme 1 shows the preparation of N-tert-butyloxycarbonyl-(E)-4-amino-2-butenoic acid (3).

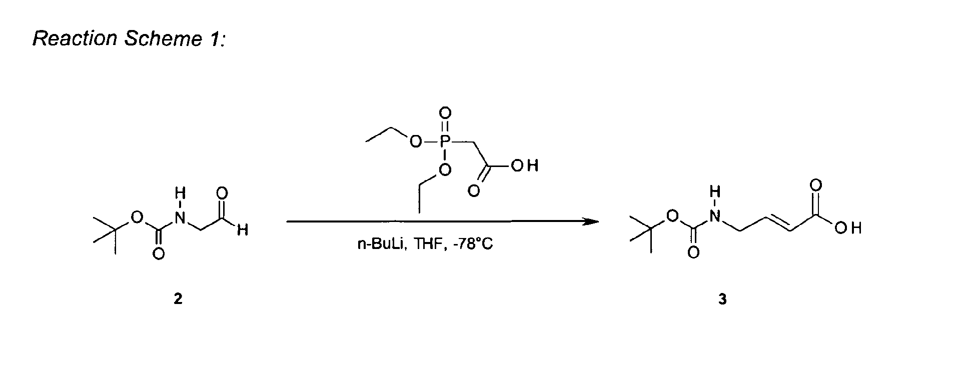

### Peptide synthesis of Ac-seq1-X-seq2 (4)

Peptides were synthesized at a 25 µmol scale using an automated multiple peptide synthesizer (Syro I from MultiSynTech, Witten, Germany). The sequences were assembled as C-terminal amide on polyoxyethylene-grafted polystyrene resin, to which the Rink amide linker was attached (TentaGel S RAM resin, 100 mg, 0.25 mmol g⁻¹). Five equivalents of Fmoc-amino acid/DIC/HOBt (0.36 M in DMF) were coupled (2 x 1 h + capping with acetic anhydride/pyridine/DMF 1 : 2 : 3) for each coupling cycle. The N-terminus was acetylated using acetic anhydride/pyridine/DMF (1 : 2 : 3, 30min).
X = Lys(Mtt), Orn(Mtt), Dpr(Mtt), or DAB(Mtt)
Dpr = 2,3-diaminopropionic acid (Fmoc-Dpr(Mtt)-OH from Nova)
DAB = 2,4-diaminobutyric acid (Fmoc-DAB(Mtt)-OH from Nova)

### Preparation of modified peptide (5)

For Mtt group cleavage from the ε-amino group of lysine (or δ-amino group of ornithine) resins were swollen in DCM and treated with 3% TFA, 5% TIPS, 92% DCM (1 ml, 8 x 20 min). The resin was then washed with DCM (1 ml), 5% DIPEA/DCM (1ml), DCM (1 ml) and DMF anhydrous (1ml). 5 eq. of *N*-tert-butyloxycarbonyl-(E)-4-amino-2-butenoic acid (3) (relative to the loading of the resin: 125 µmol, 25.2 mg) were pre-activated for 1 h with DIC (5 eq., 125 µmol, 19.4 µl) and HOBt anhydrous (5.5 eq., 137.5 µmol, 18.6 mg) in 0.5 ml DMF anhydrous. The solution was added to the resin and shaken for 5d. Peptides were cleaved from the resin as C-terminal amides using a mixture of TFA, DCM, water, and triisopropylsilane (70 : 20 : 5 : 5) for four hours, precipitated in a cold 1 : 1 mixture of tert-butylmethyl ether and cyclohexane, extracted with water, and lyophilized. Crude peptides were purified by preparative HPLC on a 250 x 10 mm NUCLEOSIL RP18 column and characterized by RP-HPLC (column: PLRP-S 150 x 2.1 mm, particle size: 3 µm; gradient: 5-65% 0.1 % TFA/acetonitrile in 0.1 % TFA/water in 20 min, flow rate: 0.25 mL min⁻¹, detection: 214 nm) with online ESI-mass spectrometry detection (LC-MS). Reaction Scheme 2 shows Mtt group cleavage and coupling of (E)-4-amino-2-butenoic acid and isolation of modified peptide (5).

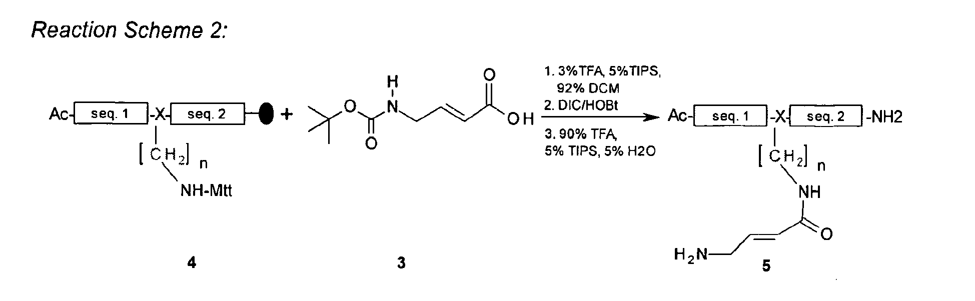

### Synthesis of modified Ubiquitin

The HA-Ub-intein-chitin-binding-domain fusion construct was expressed in E. coli DH5alpha pTyb2HAUb (DSM 24289)and induced with 0.5 mM IPTG at 20°C O/N (2L). Cell were pelleted by 3000 Xg, resuspended in 100 ml 100mM NaOAc, 50 mM HEPES pH 6.5, 100 µM PMSF and lysed by French press at 20000 psi. The suspension was clarified at 24000 Xg and incubated with 15 ml chitin beads ( New England Biolabs) for 5 h at 4 °C. Beads were loaded in column and washed with 100 ml of lysis buffer followed by 50 ml lysis buffer containing 50 m M β-mercaptoethanesulfonic acid sodium salt (MESNa). The on column cleavage for the HA-Ub₇₅ - MESNa thioester product was induced with an overnight incubation at 37°C ² . The product (2-4 mg) was eluted with 50 ml of lysis buffer and concentrated via VIVASPIN 20 -5000 MWCO (Sartorius Stedim Biotech GmbH). The recovered product was a mixture where the N- terminal (Met and) Ala were frequently split off but conducted oneself without an impact in performed labeling experiments. In contrast to the previous published method (Borodovsky et al., Chem Biol. 2002, 9:1149-59), the HA-Ub₇₅-MESNa thioester was purified to increase the yield of the chemical ligation. The purification was performed straight after the elution using ÄKTApurifier 10 (GE HealthCare) system MonoS 5/50 column, with a linear gradient from 0% to 100% B, 10 mM HEPES pH 4,5 (buffer A), 10 mM HEPES, 1 M NACI pH 4,5 (buffer B) following by desalting with Vivaspin 20 MWCO. Reaction Scheme 3 shows the preparation of HA-Ub75-MESNa (6).

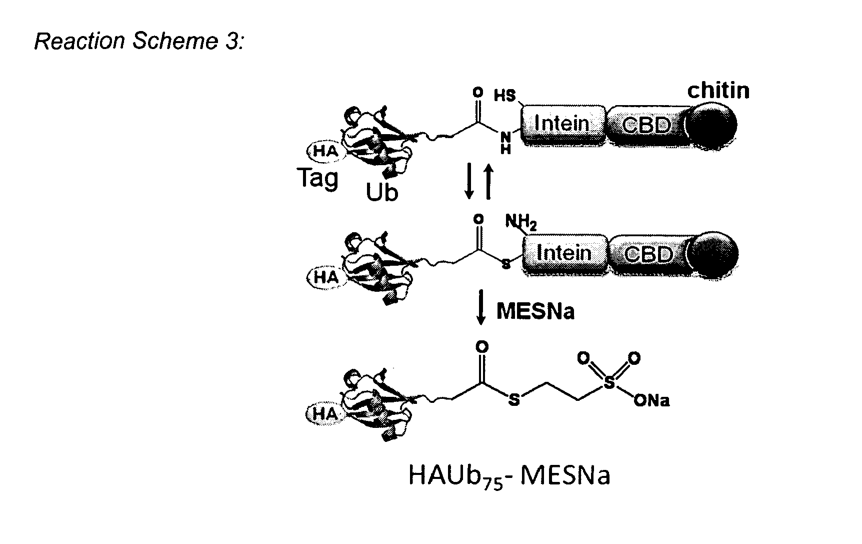

### Preparation of ubiquitin-isopeptide probe (UIPP)

Ubiquitin-iso-peptide probe (UIPP) (7) was prepared by intein-based chemical ligation. Specifically, purified HaUb₇₅- MESNa (6) in 10 mM HEPES pH 7 (2 mg in 500 µl) was mixed with 50 µl of 2M N- hydroxyl sulfosuccinimide followed by adding 12,8 mg of the modified peptide (5) as C-terminal thiol-reactive group. The solution was adjusted to pH 8 using 2 M Na₂CO₃. The ligation was performed overnight at 37 °C. The resulting products were dialyzed and concentrated to 500 µl using Vivaspin 500 -5000 MWCO (Sartorius Stedim Biotech GmbH). Usage of purified HaUb₇₅- MESNa (6) ensured higher conversion of the desired product in the ligation step. The UIPP product was characterized using nanospray time-of-flight mass spectrometer (Q-TOF 2™, Micromass) via direct injection and additional validated with Orbitrap Velos (Thermo). Reaction Scheme 4 shows the preparation of UIPP (7) by intein-based chemical ligation of C-terminal thiol-reactive modified peptide (5) with modified Ubiquitin (HAUb75 - MESNa thioester (6) ).

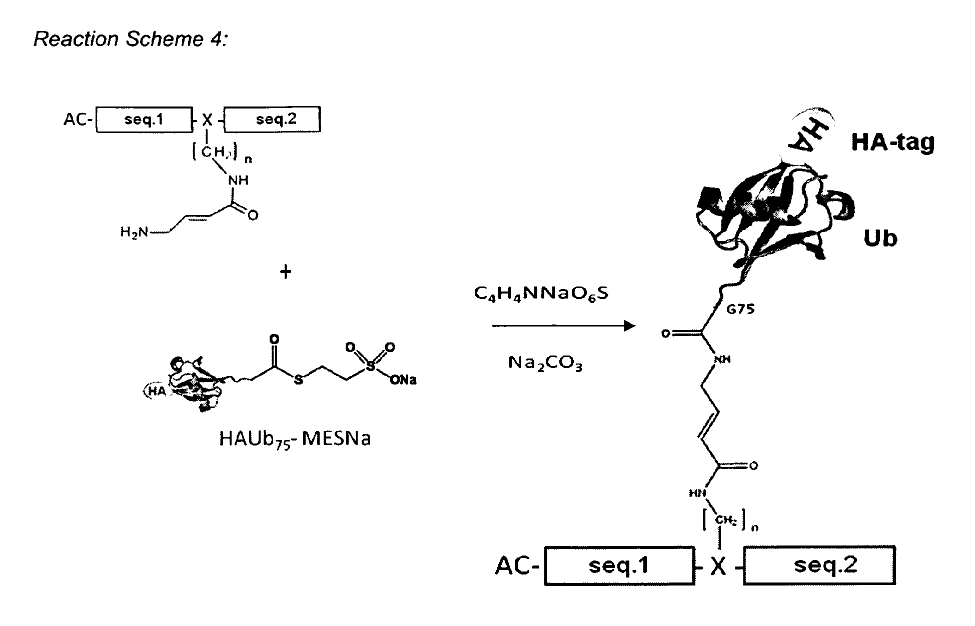

### 2. UIPP lsoPep(K48) and lsoPep(K63)

UIPP IsoPep(K48) and IsoPep(K63) where synthesized according to the above method (see item 1.). IsoPep(K48) comprises peptide sequence K48 (8): 
K48: YPYDVPDYARLIFAGK48QLEDGR
X
seq. 1 seq. 2 (8).
 lsoPep(K63) comprises peptide sequence K63 (9):

K63: YPYDVPDYARTLSDYNIQK63ESTLHLVLR
X
seq. 1
seq. 2
(9).

IsoPep(K48) resembles an ubiquitinated Lys48 site between two ubiquitins in a polyubiquitin chain, while lsoPep(K63) resembles an ubiquitinated Lys63 site between two ubiquitins in a polyubiquitin chain.

### 3. Activity-based DUB assay

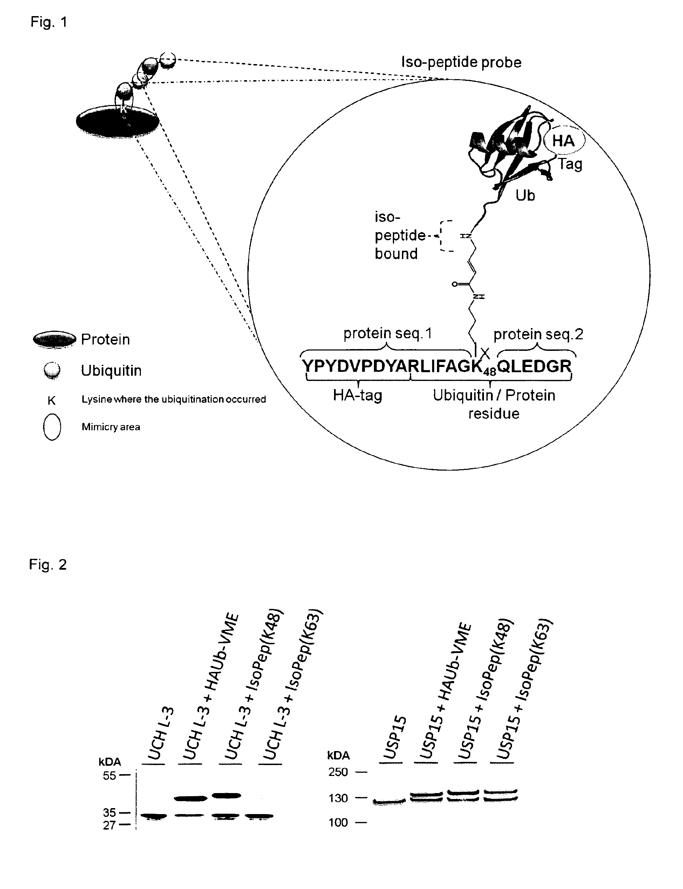

The reactivity of UIPPs can be tested in an activity-based DUB assay. The known DUBs UCH L-3 and USP15 were subjected to the commercially available HAUb-VME as a positive control and UIPPs according to the invention, namely to lsoPep(K48) comprising peptide sequence HA-Ub₄₂₋₅₄ and lsoPep(K63) comprising peptide sequence HA-Ub₅₄₋₇₂. HIS-UCH-L3 (1µM) was incubated with 1 µM probes at 37 °C for 1 h, resolved by 12 % SDS-PAGE and imunobloted with anti-HIS antibody (Penta-His,QIAGEN). Probe reactivity was investigated by UCH-L3 shift formed by probe /enzyme adducts. The same conditions were applied to test the reactivity against USP15. The successful labeling of active UCH L-3 and USP15 is demonstrated by a - 10.5 kDa shift caused by the probes (Figure 2). The isopeptide probes according to the invention caused a slightly higher shift than the standard HAUb-VME probe due to their additional peptide sequence. The probe IsoPep(K48) showed higher specificity to UCH L-3 than probe IsoPep(K63). USP15, on the other hand, was equally labeled by both probes. This is consistent with reports in the literature and shows the possibility to determine the distinct DUB specificity to certain polyubiquitin chains or ubiquitinated proteins.

## Claims

1. Ubiquitin-isopeptide-probe (UIPP) comprising a peptide sequence, **characterized in that** the sequence contains one or more residue(s) X, wherein X is represented by formula (I): 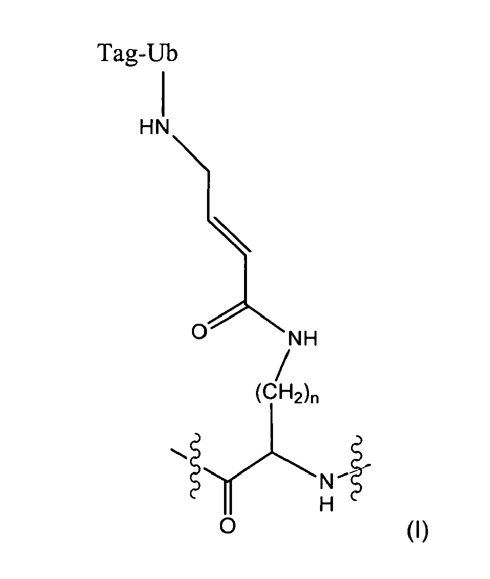 wherein
Tag is a first reporter tag that is N-terminally attached to Ub;
Ub is ubiquitin(1-75) (Ub₁₋₇₅) or a fragment of Ub₁₋₇₅ that comprises at least residues 66 to 75 of Ub₁₋₇₅; and
n is 1, 2, 3 or 4.

2. The UIPP according to claim 1, wherein the peptide sequence is derived from ubiquitin, or from a target protein with ubiquitination site.

3. The UIPP according to claim 2, wherein the target protein with ubiquitination site is selected from: E3 ubiquitin-protein ligase Mdm2, phosphatidylinositol-3,4,5-trisphosphate 3-phosphatase and dual-specificity protein phosphatase PTEN, Myc proto-oncogene protein, breast cancer type 1 susceptibility protein BRACA1, and hypoxia-inducible factor 1-alpha HIF 1α.

4. The UIPP according to claim 1 or claim 2, wherein the peptide sequence comprises a motif selected from the group: FAGXQLE, NIQXEST, IFVXTLT, LTGXTIT, ENVXAKI, ENVXAKIQD, ENVKAXIQD, ENVXAXIQD, IQDXEGI, GSDQXDLVQ, LQEEXPSSS, DCKXTIVND, DCXKTIVND, ENDDXITQ, SQEDVXEFE, DXEESVEEE, ISEXAKLEN, ISEKAXLEN, LYFTXTVEE, FKVXLYFTK, EETSEXVEN, SSPXSCAS, WSVEXRQA, TEENVXRRT, PEHLXDEVS, NTTEXRAAE, RHPEXYQGS, STEKXVDLN, PSTEXKVDL, ESNAXVADV, ENKTXGDSI, HENXTKGDS, IQNEXNPNP, EQTSXRHDS, AEDPXDLML, ESNIXPVQT, ENVFXEASS, DGEIXEDTS, ENDIXESSA, LSLXNSLND, VILAXASQE, SXQMRHQGS, STSEXAVLT, LTSQXSSEY, LSADXFEVS, STSXNKEPG, LSADXFEVS, TSKNXEPGV, VPQLXVAES, VSREXPELT, QLFTXVESE, SPNEXLQNI, AETPXPLRS, EVALKLEPN, DTEAXNPFS, TDELXTVTK, KTVTKDRME, THIHXETTS, and EQTEXSHPR.

5. The UIPP according to anyone of claims 1 to 4, wherein the peptide sequence further comprises a second reporter tag.

6. The UIPP according to anyone of claims 1 to 5, wherein the first and the second reporter tag are each and independently of each other selected from an affinity tag, biotin; or a fluorophore.

7. The UIPP according to anyone of claims 1 to 6, wherein the first reporter tag is HA-tag, His-tag, FLAG-tag, Myc-tag, biotin, or a fluorophore.

8. The UIPP according to anyone of claims 1 to 7, wherein the second reporter tag is HA-tag, His-tag, FLAG-tag, Myc-tag, biotine or a fluorophore.

9. The UIPP according to anyone of claims 1 to 8, wherein the peptide sequence is selected from the group: RLIFAGXQLEDGR (Ub₄₂₋₅₄); YPYDVPDYARLIFAGXQLEDGR (HA-Ub₄₂₋₅₄); RTLSDYNIQXESTLHLVLR (Ub₅₄₋₇₂); and YPYDVPDYARTLSDYNIQXESTLHLVLR (HA-Ub₅₄₋₇₂).

10. The UIPP according to anyone of claims 1 to 9, wherein n is 3 or 4.

11. A method for preparing an UIPP, wherein the method comprises the steps of:
(a) synthesis of a peptide that comprises at least one lysine, ornithine, 2,3-diaminopropionic acid, or 2,4-diaminobutyric acid residue, wherein the amino group in the side chain of the lysine, ornithine, 2,3-diaminopropionic acid, or 2,4-diaminobutyric acid residue is orthogonally protected by a protecting group selected from: Alloc (allyloxycarbonyl), Dde 1-(4,4-dimethyl-2,6-dioxocyclohex-1-ylidene) ethyl, ivDde (1-(4,4-dimethyl-2,6-dioxocyclohex-1-ylidene)-3-methylbutyl), Mmt (methoxytrityl), Mtt (methyltrityl), and Z (benzoyloxycarbonyl);
(b) reacting the peptide obtained in step (a) with N-tert-butyloxycarbonyl-(E)-4-amino-2-butenoic acid according to formula (II) 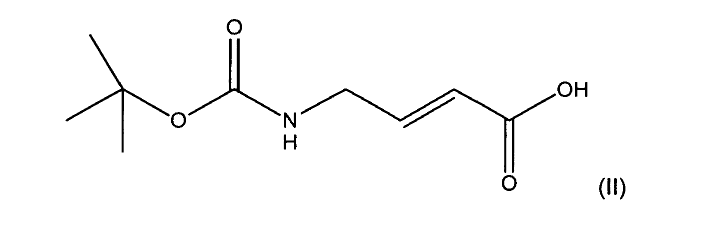 to thereby form a side chain modified peptide comprising at least one residue Y, wherein Y is represented by formula (III) 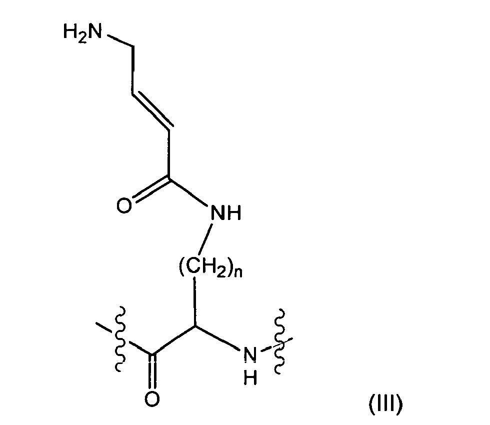 wherein n is 1, 2, 3 or 4.
(c) intein-based chemical ligation between the side chain modified peptide of step (b) and a modified ubiquitin thioester to thereby form the ubiquitin-isopeptide-probe according to the present invention.

12. The method according to claim 11, wherein the modified ubiquitin thioester used in step (c) is HA-Ub₇₅-MESNa thioester.

13. A method for isolating a deubiquitinating enzyme (DUB) from a sample containing cells or a cell extract, wherein the method comprises the steps of:
(a) treating the sample with an UIPP according to the invention; and
(b) separating DUBs.

14. The method according to claim 13, wherein step (b) is carried out using magnetic separation, immunological separation, gel filtration chromatography, affinity chromatography, column chromatography, displacement chromatography, electro chromatography, gas chromatography, high performance liquid chromatography, ion chromatography, micellar electrokinetic chromatography, normal phase chromatography, paper chromatography, reversed-phase chromatography, size exclusion chromatography, thin layer chromatography, gel electrophoresis, centrifugation, adhesion, or flow cytometry.

15. The UIPP according to anyone of claims 1 to 10 for use as a research tool.


SEQUENCE LISTING

<110>  Helmholtz Centre for Infection Research GmbH (HZI)

<120>  Novel Ubiquitin-Iso-Peptide-Probes

<130>  19293-EP

<160>  4

<170>  PatentIn version 3.5

<210>  1
<211>  75
<212>  PRT
<213>  Homo sapiens

<400>  1

Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly
65                  70                  75

<210>  2
<211>  9
<212>  PRT
<213>  Influenza A virus

<400>  2

Tyr Pro Tyr Asp Val Pro Asp Tyr Ala
1               5

<210>  3
<211>  8
<212>  PRT
<213>  Artificial Sequence

<220>
<223>  synthetic sequence

<400>  3

Asp Tyr Lys Asp Asp Asp Asp Lys
1               5

<210>  4
<211>  10
<212>  PRT
<213>  Homo sapiens

<400> 4

Glu Gln Lys Leu Ile Ser Glu Glu Asp Leu
1               5                   10